# EUROPEAN PATENT SPECIFICATION

(11) **EP 3 359 854 B1**
(45) Date of publication and mention of the grant of the patent: **02.09.2020**
(21) Application number: 16853996.3
(22) Date of filing: 07.10.2016
(51) Int. Cl.: F16K 37/00, A61M 39/22, F16K 11/085

(54) **MEDICAL VALVE**
MEDIZINISCHES VENTIL
SOUPAPE MÉDICALE

(30) Priority: 08.10.2015 SE 1551292
(43) Date of publication of application: 15.08.2018
(73) Proprietor: CYTO365 AB, 263 65 Viken (SE)
(72) Inventor: TÖRNBLOM, Micael, 26365 Viken (SE)
(86) International application number: PCT/SE2016/050968
(87) International publication number: WO 2017/061945

(56) References cited:
- EP-A1- 1 808 627
- WO-A1-2014/071209
- WO-A1-2014/071209
- CA-A1- 2 216 493
- US-A- 3 636 915
- US-A- 4 299 251

## Description

### TECHNICAL FIELD

The present inventive concept relates to medical valves, such as stopcocks, especially medical valves for administration of drug fluids and other fluids. More specifically, the inventive concept relates to a medical valve providing an optical indication of selected valve positions.

### BACKGROUND AND SUMMARY OF THE INVENTION

In medical therapy, small valves are used as stopcocks in infusion treatments such as cytostatic, antibiotics, nutrition, anesthesia, and transfusion. The stopcock may be used to control fluid in a flow or multiple flow paths. Stopcocks are continuously being developed and getting more advanced controlling more drugs that either combine or separates drugs in infusion treatments.

The handling sequence normally requires the user to turn a handle of the valve both clockwise and anticlockwise many times during use. The many positions may cause confusion to the user. In prior-art medical valves or stopcocks, an elongated handle connected to, or integrated with the rotating valve member is often used as a physical indicator for indicating one or more possible valve positions.

The handle may be shaped to give the user a clue of the correct setting, such as a T-shape. However this T-shape is only correctly illustrating the all open position, in all other positions, this shape is not optimum, resulting in that the stopcocks may be hard to understand and there is a risk of administration error. It requires both training and experience to understand even how the common three-way stopcock is to be used.

Furthermore, the many different stopcocks may look the same but the number of positions may vary between each depending of the different versions of the stopcock.

It is naturally especially important that the indication of the setting is correct when handling drugs so that a patient is not given the wrong drug due to handling errors.

WO 11011057 discloses a stopcock provided with an identification of the different valve positions. This prior-art solution requires a rather large embodiment that radially extends from the center of rotation that increase cost of material but is also a bulky structure that may be more difficult to disinfect. Furthermore, movable external parts outside the main stopcock may get broken and smaller parts may get stuck in the hollow disc and jamming the valve. It may also be a risk for the user who normally uses protective gloves to get stuck and it may result in tearing the gloves open. US 4299251 A discloses a medical rotary valve according to the preamble of claim 1.

There is thus a need for an improved medical valve with enhanced indication of different valve positions. There is especially a need for a solution which may be used within small medical valves where the available space is limited.

According to an aspect of the inventive concept, there is provided a medical valve or stopcock for use in administration of fluids, such as drug fluids, said valve comprising :
a housing, and
a valve member which is rotatably supported in the housing for rotation between a plurality of valve positions about an rotational axis,
wherein the housing is provided with an indicator means, and
wherein the rotatable valve member is provided with an optical element which is arranged to rotate together with the valve member and which is arranged to reflect images of said indicator means in said plurality of valve positions, each reflected image being visible to a user and being associated with a corresponding valve position

When handling the valve, the user when looking at the valve will be able to see images of the indicator means, referred to as indicator images in the following. These indicator images may be text, symbols, color gradients or any other type of information. These indicator images would thus be images of corresponding "objects" of the indicator means. The indicator images would typically comprise different images, such that a different image is shown for each valve position. However, some valve positions may be associated with one and the same symbol, text or the like, such as the text "STOP" for two or more closed valve positions or in another example "Saline" for multiple saline positions or in yet another example "N" for neutral saline positions and "D" for drug positions. However, such identical images would actually still be images of different portions of the indicator means.

Thus, the valve housing is provided with indication means and the optical element is arranged to be positioned in different rotational positions about the rotational axis.

In some embodiments, the housing may comprise a cylindrical or conical wall supporting the valve member, wherein the indicator means is arranged along a part of the housing wall. As a non-limiting example, the axial extension of the indicator means may be in the order of a few millimeters, such as 1-5 mm. The indicator means may be arranged inside the housing wall on the inner surface thereof or in a recess or the like on the inner surface, on the outside the housing wall or embedded or inserted into the housing wall. The indicator means may extend all the way round the housing wall or only partly.

In some embodiments, said indicator means may be formed as a separate exchangeable part attached to the housing. The indicator means may also, at least partly, be integrally formed with the housing.

In some embodiments, the valve member may be provided with a handle at a proximal end of the valve member for manually rotation of the valve member, wherein said indicator means is located axially between said handle and a more distal section of the valve where fluid flow control takes place in the valve.

In some embodiments, the optical element may be arranged such that said images are viewable by a user in the direction of the rotational axis, preferably in a direction co-axial with the rotational axis.

In some embodiments, said optical element may be arranged to provide said images as enlarged images of said indicator means.

In some embodiments, the optical element may comprise a convex image surface such that said images are viewable by a user from all viewing directions up to a semisphere centered in relation to said rotational axis.

In some embodiments, the optical element may comprise at least one reflective surface for diverging an optical view path through the optical element.

In some embodiments, the optical element may comprise a display surface facing towards the user and wherein said optical element is arranged to image said indicator means as said images on or close to said display surface. A user may then perceive the indicator means as being placed on the outer surface of the optical guide element via an optical effect due to the design of the optical element. The display surface may be a diffuse display surface.

A valve according to the inventive concept may be designed to operate by the use of ambient light only, wherein the ambient light may reach the indicator means via the view path of the user and or via other paths, such as via translucent and or transparent and or hollow parts of the housing and or valve member. However, in some embodiments the valve may further comprise at least one separate light source for illuminating the indicator means. This light source may be located such that emitting light from the light source is reflecting on the indicator object either directly or indirectly via the optical element. The external light source should preferably be located radially inside the indicator means because of the size constraints of medical valves.

In some embodiments, the optical element may comprise a first and a second optical axis, wherein the first optical axis extends at least radially from the second optical axis and wherein said second optical axis is co-axial with the rotational axis. The first optical axis may be defined by an entry surface of the optical element. The second optical axis may be defined by a display surface of the optical element. The two optical axis may be connected via an internal reflective surface of the optical element, angled 45 degrees from a rotational plane extending perpendicular to the optical axis. Optionally, this angle may be 45 degrees plus an additional angle α less than 45 degrees to improve the internal reflection. If no such additional angle α is provided (α = 0 degrees), the first and the second optical axis may be angularly separated by 90 degrees .However, if said additional angle α is provided, the first and the second optical axis may be angularly separated by 90 degrees plus an additional angle of 2α in order to get a more complete and/or more correct image of the indication on the display surface.

The optical element may be constructed such that light is guided from an exterior of said optical element to said indicator means, making the indicator means visible when the optical element is viewed from the exterior and the optical element is positioned in any of said rotational positions.

The optical element is preferably placed in the center along the rotational axis. The valve housing is preferably arranged so that it at least partly surrounds the optical element in a circumferential direction in relation to the rotational axis and where the optical element is placed vertically so that the said first optical axis intersect on the center of the indicator means.

The indicator means may comprise any type of indications depending on the application of the valve, the language and country of the user and other factors. The indicator means may comprise e.g. symbols, numbers, text, colors, color gradients, or combinations thereof, to inform the user of the present valve setting.

As an example, in a stopcock having a drug position, a saline position (neutral fluid) and a stop position (closed), the indicator means may comprise three differently colored symbols, such as a blue colored "N" for neutral fluid (saline), a red colored "D" for drug, and a black colored "S" for Stop. As an alternative, instead of letters the indicator images may be arrow symbols (optionally also colored) showing the flow path from respective inlets to the outlets of the valve.

The rotational setting, i.e. the valve position, of the valve is thus viewable through the optical element. This design will make it possible to design an optical system that is very small and could be incorporated to most stopcocks designs without need for extensive redesign of its exterior which has the favor of easy adaption for the user of previous stopcocks, and without the space consuming indications of the prior art. The indication of the indicator means may be protected from wear, since the indicator means is preferably not exposed to the surroundings, but is only facing the optical element.

The optical element may comprise at least one surface to provide reflection or internal reflection to diverge an optical view path from the indicator means towards the user. This feature will enable the use of an indicator means positioned e.g. facing in the direction towards the rotational axis.

The optical element may be arranged to enlarge the part of the indicator means that it is directed to. If the optical element is implemented as a prism, this prism may be arranged to provide said image enlargement by its shape of a lens on the object side and/or the image side of the prism, and/or an image enlarging reflective surface of the prism. Image enlargement may also be implemented by the use of one or more separate lenses. This embodiment of the present inventive concept is an advantage since it will enable the use of small indications on the indication means (in the order of a few millimeters), enabling indication images in all rotational positions, without affecting the visual experience from a distance typically but not limiting to an arm's length of the user. The smaller indications, such as text, the more text will be available for imaging.

The optical element may have a convex display surface facing towards the exterior such that the indicator images are viewable from the convex display surface from all viewing directions up to a semisphere from said rotational axis. In that way, the indicator image is viewable in all directions above the normal plane to the rotational direction so that a user may observe the indicator image with a view path other than only co-aligned with the rotational axis. A larger viewing angle interval is also beneficial when two observers look at the valve and the same indicator image at the same time.

Typically but not limited to, the inventive concept will be used for medical disposable valves that are produced in high volumes with injection molding. The total cost of injection molding production of medical valves according to the inventive concept may be significantly lowered if the indicator means is implemented as a separate or exchangeable part or module. Thereby, the same mould tool may be used for the housing even if the valve is intended for different uses and/or different countries (languages). Different indicator means (carrying different indications) may then be attached to the housing, or replaced as needed, without any need to use different mould tools.

The optical element may comprise a prism, a mirror, a mirror coating, a lens, an optical body, a one piece injection molded body, a compound lens, a compound lens with different material, and a GRIN type lens. The term "optical element" may also be considered in some embodiments to involve more than one element, such as a combination of a mirror or a prism and one or more lenses.

Any surface of the optical element facing the exterior may be provided with a transparent or diffuse or translucent protective overlay, such as a film, a coating, or any other rigid or semi-rigid material.

In some embodiments, the optical element may be movable between at least a first and second position along the rotational axis redirecting the view path to further indicator means. The user may e.g. pull or push the optical element up or down to access a new level of annular or partly annular indicator means expanding the number of settings possible.

In some embodiments, the medical valve may comprise means arranged to give a user a haptic or tactile response when the valve element is positioned at predetermined valve positions, making it easier for a user to determine the correct rotational position.

In some embodiments, the indicator symbols, text or the like of the indicator means may be visible by ambient light that illuminates the indicator means via said optical element, i.e. the light illuminating the indicator means only enters the optical element via the image side (external side) of the optical element.

In some embodiments, the indicator means may be visible by ambient light that illuminates the indicator means without entering the optical guide element from the display surface (image side). The indicator means may e.g. be illuminated via light through a transparent part in the housing body and/or the valve member and/or a through-hole in the housing and/or in the valve member.

The valve may further comprise a separate light source for illuminating the indicator mean, wherein the light source is placed so that the light either first reflects the indicator means before entering the optical element or first enter the optical element then reflects on the indicator means. The light source may e.g. be placed in either in the valve member or in the valve housing. The light source may be an electrical light source, such as an LED, but it may also be a luminescent material absorbing light when it can to illuminate the indicator means when it is darker. If the light source is electrical, a small battery may be included as a power source in case of e.g. a disposable valve.

In some embodiments, the indicator means may be luminescent, to make the indicator means viewable without a light source. If combined with an electrical light source, the luminescent indications may be viewable when power is not at hand.

The indicator means may have at least partly light reflective surface areas.

The indicator means may further have at least partly light absorbing surface areas for increasing the contrast relative other surface areas of the indicator means.

The indicator means may be semi-transparent with non-transparent indication marking or transparent with non-transparent indication marking (negative image).

The optical element may have at least one non planar surface. The optical element may further compromise at least one of an entry surface, a reflective surface and a display surface, wherein at least one of the surfaces is non-planar.

The optical element may further comprise at least one of an entry surface, a reflective surface and a display surface, wherein at least two of the surfaces are non-planar.

The optical element may compromise a display surface with a convex or a rotational elliptical shape or an asphere, and or have an entry surface with a convex or a rotational elliptical shape or an asphere shape to enable an image enlarging effect.

### BRIEF DESCRIPTION OF THE DRAWINGS

The inventive concept and advantages thereof, as well as some non-limiting embodiments and examples thereof, will now be further described with reference to the drawings, in which:
Figs 1a and 1b are a perspective view and an exploded view, respectively, of a first embodiment of a medical valve according to the inventive concept.
Fig. 1c is a top view the valve of Fig. 1a where an indicator image is shown at a display surface.
Fig. 1d is a cross-section along line A-A in Fig. 1c.
Figs 1e-h each comprises a top view, a side view and a cross-sectional view of the valve in Figs 1a-d in different valve positions, wherein different indicator images indicates the fluid path in the respective valve position.
Fig. 1i illustrates a closed position of the valve in Figs 1a-d.
Fig 2 illustrates, with a top view, a side view and a cross-sectional view, a valve according to the inventive concept having a priming or flushing valve position.
Figs 3a and 3b are perspective views of a valve comprising rotational stop means for limiting rotation of the valve member, illustrating the valve member in two end positions.
Figs 4a and 4b each comprises a top view, a side view and a cross-sectional view of a valve according to the inventive concept having one inlet only.
Figs 5a and 5b are a side view and a top view, respectively, of a medical valve according to the inventive concept comprising multiple drug inlets.
Fig. 6a is a front view of an optical element and an indicator means.
Fig. 6b is a cross-sectional view along line A-A in Fig. 6a.

### DESCRIPTION OF EMBODIMENTS

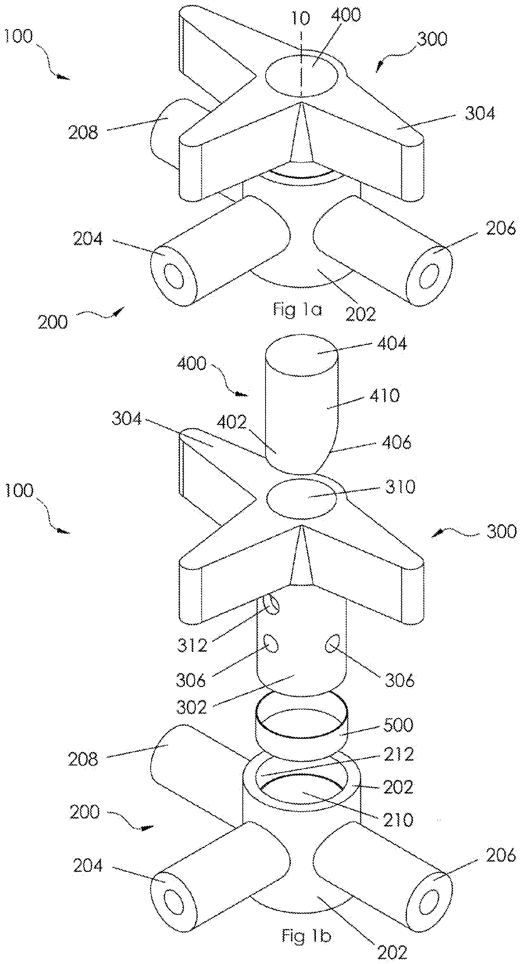

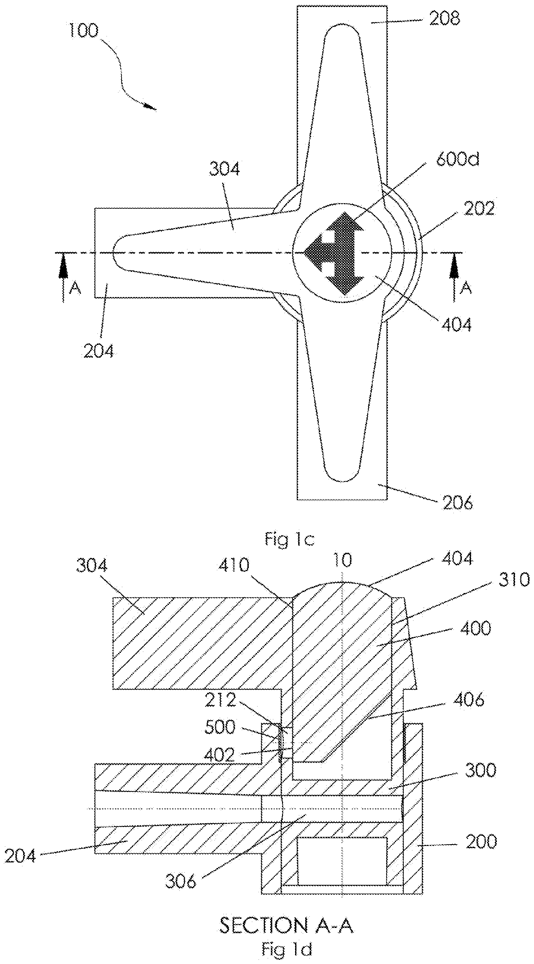

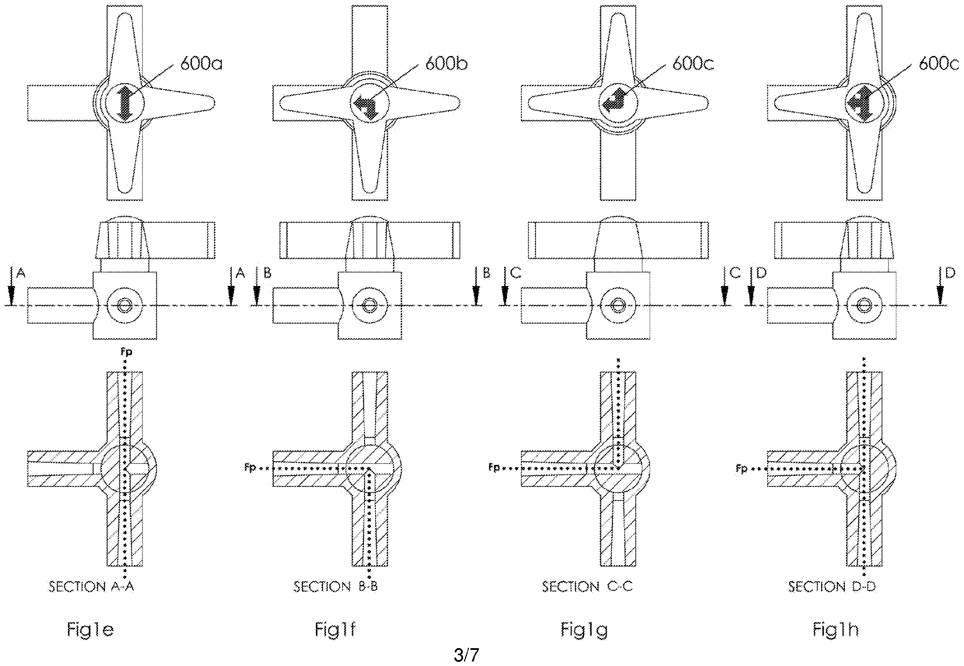

Figs 1a-d illustrate a non-limiting example of a medical stopcock or valve 100 for use in infusion treatments such as cytostatica, antibiotics, nutrition, anaesthesia and transfusion. The valve may typically be a disposable-type valve.

The valve 100 comprises a housing 200 and a valve member 300 which is rotatably supported by the housing 200 for rotation about a rotational axis 10 between a plurality of different valve positions for controlling the flow path through the valve. In some embodiments, the valve member 300 may be freely rotatable in 360 degrees. In other embodiments, the valve member 300 may be rotatable in a smaller angular interval. The term "valve positions" refers to predetermined positions in which different flow paths are established by the valve or it may refer to an all flow stop position.

The housing 200 may comprise a housing wall 202 which may be cylindrical as shown or conical. In general, the housing wall 202 is preferably rotationally symmetric relative the rotational axis 10. The valve 100 may be used to control fluid in one flow path or multiple flow paths. For this purpose, the housing 200 may have two inlet ports 204, 208 and one outlet port 206, but the number of inlet ports and outlet ports may vary. The ports may have corresponding openings in an inner cavity of the housing to be fluidly connected to openings in the valve member 300.

Common for all stopcocks or medical valves is that the fluid or fluids are controlled by means of the valve member 300. In this embodiment, the valve member 300 comprises a cylindrical portion 302 which is sealingly and rotatably engaged in the inner cavity 210 of the housing 200. The valve member 300 is provided with a handle 304 for rotating the valve member and thereby selecting valve positions. For flow control, the valve member 300 may be provided with one or more channels 306 having openings in the cylindrical portion 302 to be sealingly connected to the ports 204, 206 and 208. As in the prior art, the handle 304 may have a physical shape (T-shape in this embodiment) corresponding to the layout of inlets and outlets.

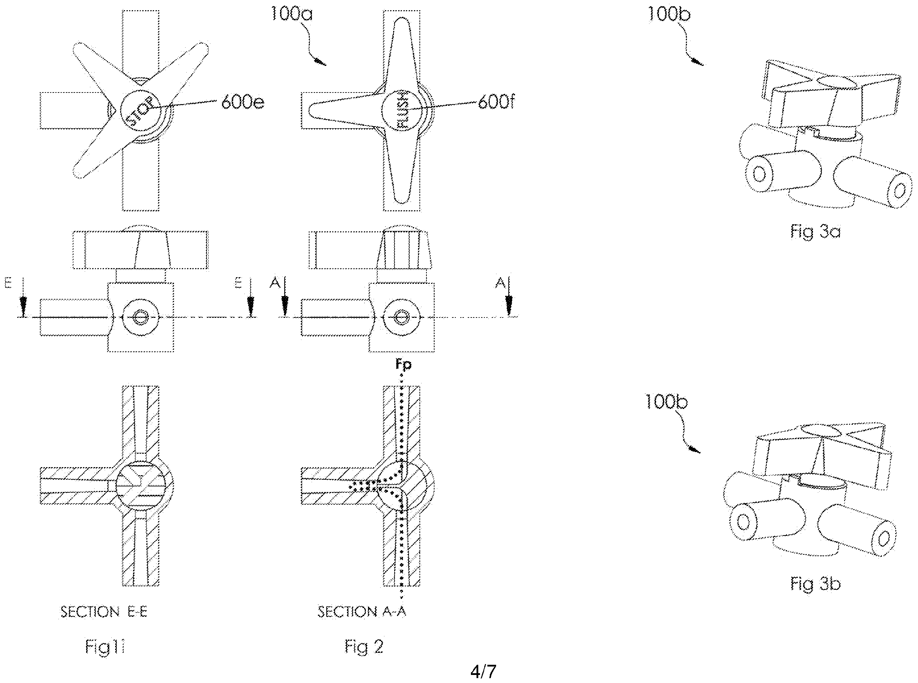

The channels 306 may provide different fluid connections between the inlets and outlets 204, 206 and 208 as illustrated in Figs 1e-1h. In each figure, the flow path Fp is indicated with a dotted line. In Fig. 1i, the valve is in a closed position.

To assist a user in operating the valve, i.e. to assist the user to understand how the handle 302 of the valve is to be positioned, the valve 100 is provided with optical means 400, 500 for providing visual indications or images 600 corresponding to the selected valve positions. These visual indications will be referred to as "images" or "indicator images". The indicator images may optionally directly visualize the selected flow path as shown in Figs 1e-h. The indicator images may optionally comprise text as shown for example in Fig. 1i ("STOP").

In the present embodiment, the optical means comprises an optical element 400 and an indicator means 500. The housing 200 is provided with the indicator means 500 and the rotating valve member 300 is provided with the optical element 400. The optical element 400 and the indicator means 500 may be formed as separate parts or units, or as an alternative one of them or both may be formed integrally with the housing 200 and the valve member 300, respectively. Thus, in this embodiment, the housing 200 and the indicator means 500 would normally be held essentially stationary in use whereas the valve member 300 and the optical element 400 would co-rotate when a new valve position is selected. In a selected valve position, a portion of the indicator means ("the object") would be visible to the user, at the top of the handle 304, as an indicator image 600.

Figs 1e-i illustrate the valve in Figs 1a-d in five different valve positions. For each valve position, a corresponding indicator image 600a-e is visible on the top of the handle 304 at the center thereof. When there is an open flow path (Figs 1e-h), the indicator image is an arrow symbol 600a-d directly illustrating the selected flow path. In the closed position (Fig. 1i), the indicator image 600e is a text message ("STOP"). These symbols and this text are thus arranged on the indicator means 500.

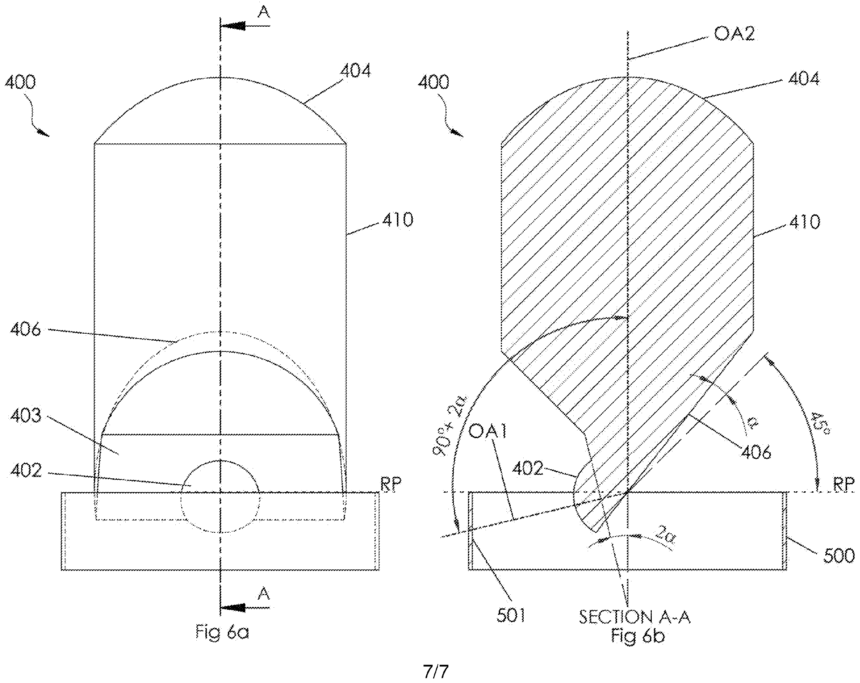

As shown in Figs 6a and 6b, the optical element 400 may as in this embodiment be an optical guide element, i.e. a solid optical element such as a prism, having an object side 402 and an image side 404. The object side 402 will be facing towards the indicator means 500 (which forms the "object" to be reflected or imaged) and will be directed towards the portion of the indicator means 500 that is to be reflected to the user, i.e. to be seen by the user. The image side 404 of the optical element 400 is visible to the user and may form a display surface at the top of the valve. The optical element 400 may also have a reflective surface 406 whereby the optical element 400 may direct, by a single reflection, a view path so that an indicator image 600 of part of the indicator means 500 may be visible at the display surface 404 to the user from above the handle 304.

Any surface of the optical element may have concave or convex shapes or any degree thereof, or flat surface, or any combination thereof such as the shape of a Fresnel lens, to achieve the optimum view for a user. The optical element may also consist comprise two or more parts made of different material and having different refractive indexes, wherein each such part may also have variation in density and variation of refractive index such as a GRIN lens.

The valve may be designed such that the indicator images are visible at the center of the handle, such that the indicator image is visible to the user when the user look at the valve within a view path that is normally directly from above colinear to the rotational axis at a distance normally up to an arm's length of the user. The user does not have to look for any other symbols outside the view path when turning the handle. This is a great advantage since the human's eyes vision uses peripheral vision and direct vision where the direct vision is limited by a small focus angle. The user does not need to move its direct vision to different location as with the prior art, but can stay focused on the displayed image. This inventive valve will give the user all information with direct feedback of the current valve position, of the rotational direction and the next selected valve position. In addition, the valve may be arranged to provide a tactile feedback to the user when the handle is positioned in any of its valve positions.

In this embodiment, the optical element 400 is located and mounted as a separate unit in an upper, central cavity 310 of the valve member 300. This space of the valve member or handle is actually often already present in today's medical valves, with the advantage that the invention may be implemented in known valves with less modification in the manufacturing. The optical element 400 may have a cylindrical or conical portion with an outer surface 410 engaging the inner surface of the central cavity 310, optionally with a press fit engagement.

The indicator means 500 may be implemented as one unit, a plurality of separate units or totally or partly integrally formed with the housing 200. The indicator means 500 would provide the "objects" to be reflected as images by the optical element 400. The indicator means 500 would normally be arranged at a radially distance from and distributed about the rotational axis 10. Especially, the indicator means 500 may extend at least partly in an axial direction parallel with the rotational axis 10. In the present embodiment, the indicator means 500 is in the form of an annular or partly annular ring 500, such as a plastic strip, extending around the inner periphery of the housing cavity 210.

The indicator means 500, such as a strip, may carry indicators such as symbols, text, colors, etc and combinations thereof. These indicators will form the objects to be seen as images via the optical element, preferably one at the time for each valve position. The objects may also be "negatives" or masks.

Each indicator may be a text or a symbol corresponding to each rotational position with contrast color, such as white background and black print, or any color or gradients of color reflecting light that is emitting from a light source outside the valve guided through the optical element, or transparent or semi-transparent material where the light is transmitting through the material of the image, or a hollow shape creating the image, even a self illuminating text or background with contrast text. In one example the indicators may be placed on the strip 500 that may be plastic and may be glued or self-adhered to the inside of the valve housing or by other means fitted on the housing so that each indication is aligned with correct position and doesn't move when the handle is turned. In another example the indicators may be attached by the same manner on the outside of the valve house with indicator facing towards the rotational axis.

The valve housing 200 may be provided with a circumferential recess 212 to make room for the plastic indicator strip 500 due to tight seal of the valve member 300 and housing 200. As shown in Fig 1d, the plastic strip 500 may preferably be located above the sealed area that holds the fluid within the valve, i.e. the axial part of the valve where the fluid flow control takes place. There is a benefit in the manufacturing process to produce valves with this recess 210 since it normally does not interfere with the basic fluid control function of the valve 100. The same valve housing 200 may be produced even if the purpose is not to use and incorporate the indicator means (strip) 500 and not use the optical identification. The dual purpose saves costs for tooling that does not need to be changed for different configurations.

The center of the stopcock handle 304 has often already a cavity 310 due to the mould process that these valves are normally produced with. There is a benefit that only small changes are needed on the handle design to incorporate this optical element 400. Many manufacturers require their product to look and feel as their own product with similar design so that the user does not need any extra education. The handle cavity 310 may be provided with a hole 312 or a transparent surface through the valve member wall revealing the valve housing inner cavity 210. The same valve member 300 and/or handle 304 may be produced even if the purpose is not to use and incorporate the indicator means 500 and not to use this optical identification. To produce one design of the valve housing saves money on the normally very expensive mould tool cost that does not need to be changed from different configurations.

Fig 6a and 6b illustrates an optical element 400 where the entry surface 402 is a convex shape in this non limiting example but could instead be a rotational elliptical shape, an asphere or a cylindrical surface or a flat surface. The entry surface 402 is defining the first optical axis OA1. The image surface 404 is defining the second optical axis OA2 which is co-axial with the rotational axis. The two optical axis are connected by a reflection surface 406 that is normally angled 45 degrees from the rotational plane RP. In this non-limiting example, reflection is achieved by total internal reflection within the prism and the surface 406 may be corrected with an additional angle of α less than 45 degrees to improve the conditions for achieving total internal reflection. The two optical axis may then preferably be angular separated with 90 degrees + 2α.

Fig. 2 illustrates an alternative embodiment of a valve 100a according to the inventive concept having a priming or flushing position (the valve position shown in Fig. 2), where a neutral fluid such as saline may prime the valve and or inlets before use to get rid of any air bubbles in the inlets, and/or flush the valve and/or inlets to rinse from drug residuals.

Figs 3a and 3b illustrate an embodiment of a valve 100b having means for limiting the rotational movement of the valve member. In this embodiment, the housing is provided with one or more rotational stops 220 which will engage with and stop the valve member during its rotation.

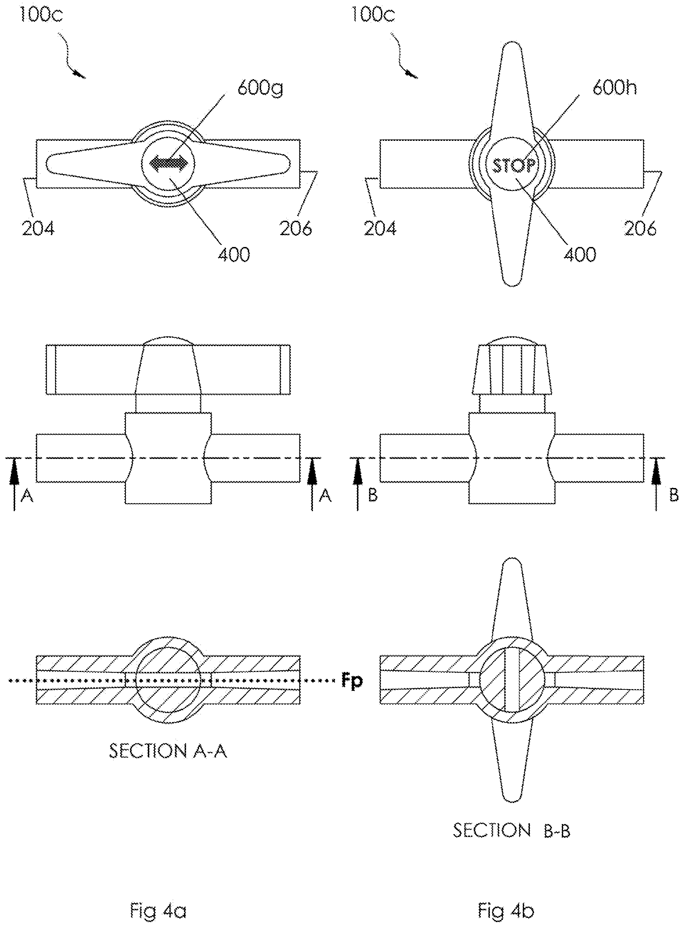

Figs 4a and 4b illustrate another embodiment of a medical valve 100c having one inlet 204 only and one outlet 206, wherein only two indications are used, one double arrow 600g and one text 600h.

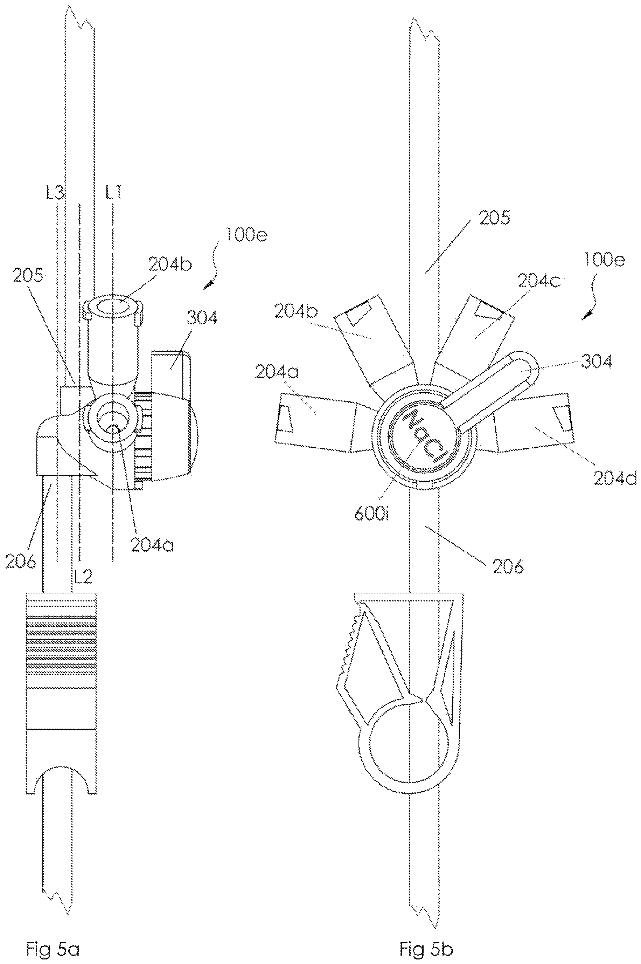

Figs 5a and 5b disclose another embodiment of a medical valve 100d according to the inventive concept provided with an optical element 300. In this embodiment, the valve 100d has multiple drug inlets 204a-d at a level L1, one neutral fluid (saline) inlet 205 at a level L2 as well as one outlet 206 at a level L3. In this embodiment, the handle 304 may preferably be aligned with the drug inlets in the respective drug positions of the valve. However, the intermediate saline positions may preferably be indicated by the present inventive concept such as shown in Fig. 5b with the indication "NaCl" 600i. Obviously, the drug positions may also be associated with optical indications as described above. The present inventive concept with an optical indication of each valve position enhances the logical sequence for the user, further enhances the feedback without the need of aligning a handle with an inlet.

The skilled person realizes that a number of modifications of the embodiments described herein are possible without departing from the scope of the invention, which is defined in the appended claims.

## Claims

1. A medical valve (100) for use in administration of fluids, such as drug fluids, comprising:
a housing (200), and
a valve member (300) which is rotatably supported in the housing (200) for rotation between a plurality of valve positions about a rotational axis (10),
wherein the housing (200) is provided with an indicator means (500), and
wherein the rotatable valve member (300) is provided with an optical element (400) which is arranged to rotate together with the valve member (300) and which is arranged to reflect images of said indicator means (500) in said plurality of valve positions, **characterized by** each reflected image being visible to a user and being associated with a corresponding valve position.

2. A medical valve (100) as claimed in any of the preceding claims, wherein said housing (200) comprises a cylindrical, conical or frusto-conical wall (202) supporting the valve member (300) and wherein the indicator means (500) is arranged along the housing wall.

3. A medical valve (100) as claimed in the preceding claim, wherein said indicator means (500) is formed as a separate exchangeable part attached to the housing (200).

4. A medical valve (100) as claimed in the preceding claim, wherein the housing (200) has an inner surface provided with a recess (212) or indication in which said indicator means (500) is arranged.

5. A medical valve (100) as claimed in any of the preceding claims, wherein said valve member (300) is provided with a handle (304) at a proximal end of the valve member (300) for manually rotation of the valve member (300), and wherein said indicator means (500) is located axially between said handle (304) and a more distal section of the valve (100) where fluid flow control takes place in the valve (100).

6. A medical valve (100) as claimed in any of the preceding claims, wherein the optical element (400) is arranged such that said images are viewable by a user in the direction of the rotational axis (10), preferably in a direction co-axial of the rotational axis (10).

7. A medical valve (100) as claimed in any of the preceding claims, wherein said optical element (400) is arranged to provide said images as enlarged images of said indicator means (500).

8. A medical valve (100) as claimed in any of the preceding claims, wherein the optical element (400) comprises a convex image surface (404) such that said images are viewable by a user from all viewing directions up to a semisphere centered in relation to said rotational axis (10).

9. A medical valve (100) as claimed in any of the preceding claims, wherein the optical element (400) comprises at least one reflective surface for diverging an optical view path through the optical element (400).

10. A medical valve (100) as claimed in any of the preceding claims, wherein said optical element (400) comprises a display surface facing towards the user and wherein said optical element (400) is arranged to image said indicator means (500) as said images on or close to said display surface.

11. A medical valve (100) as claimed in any of the preceding claims, further comprising at least one separate light source for illuminating the indicator means (500), wherein said light source being located such that emitting light from the light source can enter the optical element from other than the display surface.

12. A medical valve (100) as claimed in any of the preceding claims, wherein said optical element (400) comprises a reflective surface angled 45 degrees from a rotational plane perpendicular to the rotational axis (10), optionally plus an additional angle α less than 45 degrees, wherein said optical element (400) further comprises a first and a second optical axis (OA1, OA2), wherein said first and second optical axis (OA1, OA2) are angularly separated by 90 degrees optionally with an additional angle of 2α, wherein said second optical axis (OA2) is co-axial with the rotational axis (10).

## Patentansprüche

1. Medizinisches Ventil (100) zur Verwendung bei der Verabreichung von Flüssigkeiten, wie beispielsweise Arzneimittelflüssigkeiten, umfassend:
ein Gehäuse (200) und
ein Ventilelement (300), das in dem Gehäuse (200) zur Drehung zwischen mehreren Ventilstellungen drehbar um eine Rotationsachse (10) gelagert ist,
wobei das Gehäuse (200) mit einem Anzeigemittel (500) versehen ist und
wobei das drehbare Ventilelement (300) mit einem optischen Element (400) versehen ist, das vorgesehen ist sich zusammen mit dem Ventilelement (300) zu drehen und vorgesehen ist Bilder des Anzeigemittels (500) in mehreren Ventilstellungen zu reflektieren, **dadurch gekennzeichnet, dass** jedes reflektierte Bild für einen Benutzer sichtbar ist und einer entsprechenden Ventilstellung zugeordnet ist.

2. Medizinisches Ventil (100) nach einem der vorhergehenden Ansprüche, wobei das Gehäuse (200) eine zylindrische, konische oder kegelstumpfförmige Wand (202) umfasst, die das Ventilelement (300) stützt, und wobei das Anzeigemittel (500) entlang der Gehäusewand angeordnet ist.

3. Medizinisches Ventil (100) nach dem vorhergehenden Anspruch, wobei das Anzeigemittel (500) als separates austauschbares Teil ausgebildet ist, das an dem Gehäuse (200) angebracht ist.

4. Medizinisches Ventil (100) nach dem vorhergehenden Anspruch, wobei das Gehäuse (200) eine Innenfläche aufweist, die mit einer Aussparung (212) oder Anzeige versehen ist, in der das Anzeigemittel (500) angeordnet ist.

5. Medizinisches Ventil (100) nach einem der vorhergehenden Ansprüche, wobei das Ventilelement (300) an einem proximalen Ende des Ventilelements (300) mit einem Griff (304) zum manuellen Drehen des Ventilelements (300) versehen ist und wobei das Anzeigemittel (500) axial zwischen dem Griff (304) und einem distalen Abschnitt des Ventils (100) angeordnet ist, wo eine Flüssigkeitsströmungssteuerung im Ventil (100) stattfindet.

6. Medizinisches Ventil (100) nach einem der vorhergehenden Ansprüche, wobei das optische Element (400) so angeordnet ist, dass die Bilder durch einen Benutzer in Richtung der Rotationsachse (10) sichtbar sind, vorzugsweise in einer Richtung die mit der Rotationsachse (10) koaxial ist.

7. Medizinisches Ventil (100) nach einem der vorhergehenden Ansprüche, wobei das optische Element (400) vorgesehen ist die Bilder als vergrößerte Bilder des Anzeigemittels (500) anzuzeigen.

8. Medizinisches Ventil (100) nach einem der vorhergehenden Ansprüche, wobei das optische Element (400) eine konvexe Bildoberfläche (404) umfasst, so dass die Bilder durch einen Benutzer aus allen Blickrichtungen bis zu einer in Bezug auf die Rotationsachse (10) zentrierten Halbkugel sichtbar sind.

9. Medizinisches Ventil (100) nach einem der vorhergehenden Ansprüche, wobei das optische Element (400) mindestens eine reflektierende Oberfläche zum Ablenken eines optischen Sichtweges durch das optische Element (400) umfasst.

10. Medizinisches Ventil (100) nach einem der vorhergehenden Ansprüche, wobei das optische Element (400) eine dem Benutzer zugewandte Anzeigefläche umfasst und wobei das optische Element (400) vorgesehen ist das Anzeigemittel (500) als Bilder auf oder in der Nähe der Anzeigefläche abzubilden.

11. Medizinisches Ventil (100) nach einem der vorhergehenden Ansprüche, ferner umfassend mindestens eine separate Lichtquelle zum Beleuchten des Anzeigemittels (500), wobei die Lichtquelle so angeordnet ist, dass von der Lichtquelle abgestrahltes Licht von wo anders als von der Anzeigefläche in das optische Element eindringen kann.

12. Medizinisches Ventil (100) nach einem der vorhergehenden Ansprüche, wobei das optische Element (400) eine reflektierende Oberfläche umfasst, die um 45 Grad von einer Rotationsebene senkrecht zur Rotationsachse (10) geneigt ist, gegebenenfalls zuzüglich eines zusätzlichen Winkels α von weniger als 45 Grad, wobei das optische Element (400) ferner eine erste und eine zweite optische Achse (OA1, OA2) umfasst, wobei die erste und zweite optische Achse (OA1, OA2) um 90 Grad, wahlweise mit einem zusätzlichen Winkel von 2α winkelmäßig getrennt sind, wobei die zweite optische Achse (OA2) koaxial zur Rotationsachse (10) ist.

## Revendications

1. Soupape médicale (100) pour utilisation dans l'administration de fluides, tels que des fluides médicamenteux, comprenant :
un boîtier (200), et
un élément de soupape (300) qui est supporté en rotation dans le boîtier (200) pour rotation entre une pluralité de positions de soupape autour d'un axe de rotation (10),
dans lequel le boîtier (200) est pourvu d'un moyen indicateur (500), et
dans lequel l'élément de soupape rotatif (300) est pourvu d'un élément optique (400) qui est agencé de manière à tourner avec l'élément de soupape (300) et qui est agencé de manière à refléter des images dudit moyen indicateurs (500) dans ladite pluralité de positions de soupape, **caractérisé par**
chaque image réfléchie étant visible pour un utilisateur et étant associée à une position de valve correspondante.

2. Soupape médicale (100) selon l'une quelconque des revendications précédentes, dans lequel ledit boîtier (200) comprend une paroi (202) cylindrique, conique ou tronconique supportant l'élément de soupape (300) et dans lequel le moyen indicateur (500) est disposé le long de la paroi du boîtier.

3. Soupape médicale (100) selon l'une quelconque des revendications précédentes, dans lequel ledit moyen indicateur (500) est formé comme une partie échangeable séparée fixée au boîtier (200).

4. Soupape médicale (100) selon l'une quelconque des revendications précédentes, dans lequel le boîtier (200) a une surface intérieure pourvue d'un évidement (212) ou d'une indication dans laquelle ledit moyen indicateur (500) est disposé.

5. Soupape médicale (100) selon l'une quelconque des revendications précédentes, dans lequel ledit élément de soupape (300) est pourvu d'une poignée (304) à une extrémité proximale de l'élément de soupape (300) pour faire tourner manuellement l'élément de soupape (300), et dans lequel ledit moyen indicateur (500) est situé axialement entre ladite poignée (304) et une section plus distale de la soupape (100) où le contrôle de débit du fluide a lieu dans la soupape (100).

6. Soupape médicale (100) selon l'une quelconque des revendications précédentes, dans lequel l'élément optique (400) est agencé de telle sorte que lesdites images sont visibles par un utilisateur dans la direction de l'axe de rotation (10), de préférence dans une direction co-axiale de l'axe de rotation (10).

7. Soupape médicale (100) selon l'une quelconque des revendications précédentes, dans lequel ledit élément optique (400) est agencé de manière à fournir lesdites images sous forme d'images agrandies dudit moyen indicateur (500).

8. Soupape médicale (100) selon l'une quelconque des revendications précédentes, dans lequel l'élément optique (400) comprend une surface d'image convexe (404) telle que lesdites images sont visibles par un utilisateur de toutes les directions de visualisation jusqu'à une demi-sphère centrée par rapport audit axe de rotation (10).

9. Soupape médicale (100) selon l'une quelconque des revendications précédentes, dans lequel l'élément optique (400) comprend au moins une surface réfléchissante pour faire diverger une trajectoire de vision optique à travers l'élément optique (400).

10. Soupape médicale (100) selon l'une quelconque des revendications précédentes, dans lequel ledit élément optique (400) comprend une surface d'affichage tournée vers l'utilisateur et dans lequel ledit élément optique (400) est agencé de manière à imager ledit moyen indicateur (500) en tant que lesdites images sur ou à proximité de ladite surface d'affichage.

11. Soupape médicale (100) selon l'une quelconque des revendications précédentes, comprenant en outre au moins une source de lumière distincte pour éclairer le moyen indicateur (500), ladite source de lumière étant située de telle sorte que la lumière émise par la source de lumière puisse pénétrer dans l'élément optique depuis une surface autre que la surface d'affichage.

12. Soupape médicale (100) selon l'une quelconque des revendications précédentes, dans lequel ledit élément optique (400) comprend une surface réfléchissante inclinée à 45 degrés par rapport à un plan de rotation perpendiculaire à l'axe de rotation (10), optionnellement plus un angle supplémentaire α inférieur à 45 degrés, dans lequel ledit élément optique (400) comprend en outre un premier et un deuxième axe optique (OA1, OA2), dans lequel lesdits premier et deuxième axes optiques (OA1, OA2) sont angulairement séparés de 90 degrés, optionnellement avec un angle supplémentaire de 2α, dans lequel ledit deuxième axe optique (OA2) est coaxial avec l'axe de rotation (10).
